# EUROPEAN PATENT SPECIFICATION

(11) **EP 4 217 056 B1**
(45) Date of publication and mention of the grant of the patent: **05.08.2026**
(21) Application number: 21777811.7
(22) Date of filing: 23.09.2021
(51) Int. Cl.: A61N 2/02, A61N 2/00

(54) **STIMULATION DEVICE**
STIMULATIONSVORRICHTUNG
DISPOSITIF DE STIMULATION

(30) Priority: 23.09.2020 CH 12102020; 19.10.2020 CH 13462020
(43) Date of publication of application: 02.08.2023
(73) Proprietor: Stimit AG, 2503 Biel (CH)
(72) Inventor: MÜLLER-BRUHN, Ronja, 5210 Windisch (CH); DEGEN, Thomas, 8903 Birmensdorf Zürich (CH); FENGELS, Dirk, 6037 Root (CH)
(74) Representative: Ruttensperger Lachnit Trossin Gomoll
(86) International application number: PCT/EP2021/076269
(87) International publication number: WO 2022/063931

(56) References cited:
- EP-B1- 3 355 987
- WO-A1-2019/150378
- CA-A1- 3 088 298
- US-A1- 2012 016 280
- US-A1- 2014 249 355
- US-A1- 2018 280 711
- US-B2- 10 596 384
- G H MILLS ET AL: "Unilateral magnetic stimulation of the phrenic nerve.", THORAX, vol. 50, no. 11, 1 November 1995 (1995-11-01), GB, pages 1162 - 1172, XP055560423, ISSN: 0040-6376, DOI: 10.1136/thx.50.11.1162

## Description

### Technical Field

The present invention relates to a stimulation device for stimulating the Phrenic nerves in a human or animal body using an electric, magnetic or electro-magnetic field, thereby activating a target tissue and particularly a diaphragm in the human or animal body.

### Background Art

In medicine, it is known that for many purposes it is beneficial to activate a target tissue of a patient using stimulation by electric or electro-magnetic fields. For achieving such activation of tissues in a patient's body, it is known to directly stimulate the tissue or to indirectly activate the tissue via stimulation of specific parts of the neural system. For example, the target tissue being a muscular tissue can be activated by providing electric pulses directly to the muscular tissue or to nerves associated to the muscular tissue.

Particularly in critical care units of hospitals or other medical environments, it may be desired to activate the diaphragm of ventilated patients in order to prevent drawbacks of disuse of the diaphragm, e.g. during mechanical ventilation. It was shown that disuse atrophy of diaphragm muscle fibres occurs already in the first 18-69 hours of mechanical ventilation, and the muscle fibre cross-sections decreased by more than 50% in this time. Thus, it may be aimed uphold the functioning of the diaphragm, or to activate the diaphragm at least during the weaning period to support effective restoration of independent respiratory function. Diaphragm activation could also be used to restore "breathing pump" function in anaesthetized or sedated patients, thus activate the diaphragm regularly.

For example, US 2016/0310730 A1 describes an apparatus for reducing ventilation induced diaphragm disuse in a patient receiving ventilation support from a mechanical ventilator. The apparatus includes an electrode array of first and second types and comprises a plurality of electrodes configured to stimulate a Phrenic nerve of the patient. At least one controller identifies a type of electrode array and generates a stimulus signal for stimulating a Phrenic nerve of the patient based upon the identity of the electrode type.

In another medical field, DE 10 2007 013531 A1 describes a magnet coil arrangement with at least two coil areas that are juxtaposed and largely mirror-symmetrical. The at least two coil areas are formed in a de-central manner for producing de-centralized fields in order that the electric field with highest strength being arranged close to the edge of the magnet coil arrangement.

Further prior art is disclosed by CA 3088298 A1 and US 2012/016280 A1.

However, when two target nerves inside the body like the two Phrenic nerves shall be stimulated, the two coils shall not only be located in a close distance to each other but also operated simultaneously. Time-synchronous stimulation of both nerves has advantages to allow time-synchronous activation of both nerves thus balanced contraction of both diaphragm hemispheres. Time-synchronous stimulation of both nerves has further advantages to minimize electro-magnetic far-fields which is only possible when current in both coils is induced in exact timely synchrony and when the induced time-variant magnetic fields which oppose each other in direction. Further, when stimulating the two Phrenic nerves particularly at a neck of a patient space is comparably limited such that it is desired to have comparably small coils generating a sufficient electric or electro-magnetic field. Furthermore, current stimulator-coil solutions are not power effective, because typically one stimulator induces current in one stimulator. The coil winding systems in today's stimulators used for electro-magnetic stimulation have significant limitations. They are usually built to induce current in one coil unit having single coil or multi-coil winding in one housing.

Therefore, there is a need for a non-invasive stimulation device or a ventilation machine having such stimulation device, which allows an efficient stimulation of the two Phrenic nerves, overcome space constraints, preferably avoid co-stimulation effects of tissue in the vicinity of the nerves and are simple to apply at an appropriate location at the body.

### Disclosure of the Invention

According to the invention this need is settled by the stimulation device as defined by the features of claim 1. Preferred embodiments are subject of the dependent claims.

In one aspect, the invention is a stimulation device to stimulate a first Phrenic nerve and a second Phrenic nerve in a human or animal body for activating a target tissue in the human or animal body such as, in particular, a diaphragm. The stimulation device comprises a first coil unit configured to be positioned at the human or animal body to stimulate the first Phrenic nerve, and a second coil unit configured to be positioned at the human or animal body to stimulate the second Phrenic nerve. Each of the first and second coil units comprises at least two coil windings being wound with a variable radius into a spiral and preferably having at least one essentially straight-lined side. The at least two coil windings of each of the first and second coil are stacked on top of each other.

The term "position" and derivations thereof as used herein typically relate to a location and orientation of an element or component. If an element or component is positioned to be capable of doing something it advantageously is located and orientated to achieve the respective function. The first and second coil units being positioned to stimulate the Phrenic nerves relates to the first and second coil units being located and oriented such that the Phrenic nerves are within the electric or electro-magnetic field the respective coil unit generates.

The spiral having a variable radius may be in a flattened form such as pancake, or in form of a spherical helical such as a conic spiral, e.g., with Archimedean spiral as floor plan. In the latter case, the spiral can be still seen as a pancake but has a surface with a curvature, especially in the centre. In any event, the coil winding according to the invention has a variable radius and, thus, is non-cylindrical even when being-non-flat.

The term "spiral" as used herein may relate to any geometric form on a plane that winds around a fixed center point at an increasing or decreasing distance from the point. The form may be a continuous curve, such that a circular spiral results, continuous straight lines such that a polygonal, i.e. triangular, square, pentagonal, hexagonal or similar, spiral results, or any combination thereof. Spiral may also relate to a three-dimensional form that turns around an axis at a varying distance while moving parallel to the axis.

The term "straight-lined side" in the context of the invention relates to a side being non-round or not bent. In particular, whereas usually a circular spiral has a continuously round or bent periphery, the spiral of the coil windings of the stimulation device may have at least one essentially straight peripheral side.

Such straight-lined side of the coil windings and, thus, of the respective coil unit allows for arranging the coil unit in an appropriate alignment and location at the neck of a patient. It can be also easily tilted or rotated by the straight-lined side of the coil winding. Moreover, such straight-lined side allows for generating an electric or electro-magnetic field suitable to specifically stimulate one of the Phrenic nerves. The centre of the stimulation may be provided close to the edge of the coil winding and, thus increasing focality at this edge thus collateral stimulation of other tissue at the neck can be minimized or even prevented. If further focality is needed, the coil could be tilted as necessary to stimulate only with one edge. In particular, the straight-lined side is particularly useful for Phrenic nerve stimulation since the electro-magnetic field generated thereby may have more electric field gradient compared to conventional round coils. The centre of the stimulation may be provided close to the edge of the coil winding and, thus, closer to a neck of the human or animal being. It allows to shift the electric or electro-magnetic field to one side of the coil so that space constraints at the neck can be solved. For example, if the straight line or edge of the coil, e.g. the straight-line side of a drop or D shaped, points towards the front of the neck, the focal point of the point is closer to the straight line. Thus, the coil can be moved further backward compared to a round coil, the space consuming round side points backwards. Accordingly, the front side of the coil is space optimised and a touching of coil and tracheotomy can be avoided. A concentration of the focality to one coil side leading to collateral stimulation of other tissue at the neck can be minimized or even prevented.

By stacking the at least two coil windings of each coil unit on top of each other, it can be achieved that a comparably strong electric or electro-magnetic field is created without requiring a lot of space. In particular, for a given strength or intensity of an electric or electro-magnetic field required, the stacked design of the at least two coil winding allow for an essentially more compact construction compared to a non-stacked coil winding. Generally, the coil size can be minimized for a given induction of a stimulator with a stacked design, while still allowing the magnetic field to penetrate deeply into the body at the centre of the coil. Similar to a cylindric-type coil design a homogenous magnetic field at the centre of the coil can be created, and similar to a flat-type coil design the wires are close to the skin. Hence, using the stacked coils, both advantages can be optimised.

Therefore, the coil units of the stimulation device according to the invention are particularly suitable and efficient to stimulate the Phrenic nerves at the neck of the patient.

Preferably, each of the at least two coil windings of each of the first and second coil units may be D-shaped, teardrop-shaped or has two straight-lined sides. Such shapes of the coil windings allow for efficiently implementing and applying the coil units.

Preferably, the two straight-lined sides of the at least two coil windings of each of the first and second coil units extend in a substantially parallel direction. In particular, in application of the stimulation device, the two straight-lined sides preferably extend along the neck of the patient or along an axis of the neck. If needed, the angle of the two straight-lined sides can also be in a range of 0° to 30° to each other.

Preferably, the two coils are positioned mirror-inverted to each other. This is in favour of generating a homogenic magnetic field by the two coils.

Preferably, the two straight-lined sides or forward faces of each of the at least two coil windings of each of the first and second coil units are parallel to each other. In case of need, the angle of the two straight-lined sides or the two coil forward faces can also be in a range of 0° to 30° to each other.

Preferably, the at least two coils windings of the first coil unit are wound in a first direction, the at least two coil windings of the second coil unit are wound in a second direction, and the first direction is opposite to the second direction. Thereby, the at least two coil windings of each of the first and second coil units are wound in the identical direction but the coils face each other so that if looking from one side both winding rotations oppose each other. For example, the two coil windings of the first coil unit can be wound clock-wise and the at least two coil windings of the second coil unit anti-clock-wise. Thus, the magnetic field direction of the one coil points in the opposite direction of the other coil when the forward faces of the coils are essentially parallel and oppose each other. Such windings allow for generating the electric or electro-magnetic fields to stimulate the two Phrenic nerves, e.g. at the neck, in an efficient and beneficial manner. For example, disturbances by the two electric or electro-magnetic fields generated at the same time may be reduced because the magnetic fields opposing each other cancel out each other minimising far-field magnetic field exposure. The near field is not impacted significantly by the respective other coil. That is the stimulation process can be maintained.
The electro and magnetic field strength decrease when the distance from the source increases. Usually, a far field relates to the electro and/or magnetic field with a distance to the coil that is approximately more than 10 times of dimension of the coil. In comparison a near field relates to the electro-magnetic field close to the coil. In the present invention, the far field describes the electro-magnetic field that may negatively interfere the other medical devices arrange around the stimulation device, whereas the near filed relates to the electro-magnetic field effectively used for stimulation of the nerves. Hence, the electro-magnetic field in the far field should be kept as small as possible in order to avoid the impact to the other medical devices during the stimulation. In the present invention, the near field can have a distance of 0.1 cm, 0.5 cm, or up to 4 cm to the coil, whereas the far field can have a distance of 30 cm or 45 cm to the coil.

Preferably, the at least one straight-lined side has a length that is greater than a half of the equivalent spherical diameter of the at least two coil windings. Straight-lined sides of that size can be sufficiently large to allow an efficient implementation of the invention.

Preferably, each of the first and second coil units comprises a housing for embracing the respective at least two coil windings. In this case, the at least two coil windings may be form-fit with the housing. Such housings allow for protecting the coil windings and for a safe and convenient handling.

Preferably, each housing of the first and second coil units comprises a spiral isolation member configured to hold the respective at least two coil windings and to electrically isolate the respective at least two coil windings from each other. Such isolation member allows for ensuring integrity of the coil units. Also it can be beneficial for winding the litz wires to a predefined form such as two stacked spirals having a straight-lined side.

Preferably, the at least two coil windings of the first and second coil units are electronically connected with each other in series. This facilitates the easy control of the stimulation, especially when stimulations at the first and second Phrenic nerve need to be activation simultaneously.

A time-synchronous stimulation of both nerves has advantages to allow time-synchronous activation of both nerves thus balanced contraction of both diaphragm hemispheres. The time-synchronous stimulation of both nerves has further advantages to minimize an electro-magnetic far field which is only possible when current in both coils is induced in exact timely synchrony and when the induced time-variant magnetic fields which are at its high exactly at the same time oppose each other in direction, or almost oppose each other within +/- 20°.

Preferably, the first and the second coil units are individually positionable at the human or animal body. The term "individually positionable" is directed to placing the coil individually at the body. Thus, the coil units are not commonly positioned at one single location at the body but at two locations separate from each other. However, the coil units can be individually positionable but still being fixed to each other. Or, the coil units can, at least to a certain extent, be movable relative to each other.

The stimulation device can comprise a holder structure for holding the coil units. In particular, the holder structure may have a brace or a similar element to which the coil units are fixedly or displacably mounted.

Preferably, the first and second coil units are arranged axial or substantially axial. In conjunction with the coil windings of each coil being wound in opposing directions, two electro-magnetic fields generated by the two coil units would be capable of entirely compensating each other. In other words, the sum of the electro-magnetic fields generated by the first and second coil units would be about zero at a position having distance of 40 cm to the first or second coil unit.

Preferably, the stimulation device further comprises a control unit configured to initiate the stimulation in accordance with a predefined security policy including conditions to activate or prevent the stimulation. This security measure can be particularly useful for reducing or avoiding impact on the surrounding other medical devices, by the electro-magnetic fields generated for stimulation.

Preferably, the at least two coil windings of each of the first and second coil units are formed by the same litz wire. Thereby, the at least two coil windings in the first coil unit are electric embodied in series. The same applies to the coil windings in the second coil unit. Moreover, the first and second coil units may be also electrically connected in series.

Preferably, the at least two coil windings of each of the first and second coil units have an essentially identical form. This facilitates to stack the coil windings on top of each other and to arrange the coil windings in the housing of the coil unit.

Preferably, each of the at least two coil windings of each of the first and second coil units is essentially flat. Alternatively or additionally, each of the at least two coil windings of each of the first and second coil units can be non-flat such as conical or bent, where the latter are more convenient for the patient when placing it at the neck.

Preferably, the stimulation device is configured such that each of the first and second coil units generates biphasic impulses.

Preferably, each of the at least two coil windings of each of the first and second coil units is bent in a radius between 4 cm and 20 cm, or between 6 cm and 12 cm.

Preferably, the stimulation device is configured to generate time-variant currents in the first and second coil units being in timely synchrony.

Preferably, the stimulation device comprises a stimulator unit configured to induce currents in the first and second coil units.

Preferably, the stimulation device is configured such that an induced current in the first coil unit is the same as an induced current in the second coil unit.

Preferably, the stimulation device is configured to induce impulses generated by one current.

Preferably, the first and second coil units have two forward faces essentially opposing each other or opposing each other in an angle of +-20° or less.

Preferably, the first and second coil units have two forward faces opposing each other +/- 20°.

### Brief Description of the Drawings

The stimulation device according to the invention are described in more detail hereinbelow by way of an exemplary embodiment and with reference to the attached drawings, in which:
- Fig. 1: shows a schematic view of a stimulation device according to the invention;
- Fig. 2: shows a schematic view of the coil windings to be placed at the neck of a patient, according to the invention;
- Fig. 3a to 3c: show exemplary form of the coil windings according to the invention;
- Fig. 4a to 4b: show the housing of the coil unit according to the invention; and
- Fig. 5a to 5c: show exemplary arrangement of the coil windings in the housing according to the invention.

### Description of Embodiments

In the following description certain terms are used for reasons of convenience and are not intended to limit the invention. The terms "right", "left", "up", "down", "under" and "above" refer to directions in the figures. The terminology comprises the explicitly mentioned terms as well as their derivations and terms with a similar meaning. Also, spatially relative terms, such as "beneath", "below", "lower", "above", "upper", "proximal", "distal", and the like, may be used to describe one element's or feature's relationship to another element or feature as illustrated in the figures. These spatially relative terms are intended to encompass different positions and orientations of the devices in use or operation in addition to the position and orientation shown in the figures. For example, if a device in the figures is turned over, elements described as "below" or "beneath" other elements or features would then be "above" or "over" the other elements or features. Thus, the exemplary term "below" can encompass both positions and orientations of above and below. The devices may be otherwise oriented (rotated 90 degrees or at other orientations), and the spatially relative descriptors used herein interpreted accordingly. Likewise, descriptions of movement along and around various axes include various special device positions and orientations.

To avoid repetition in the figures and the descriptions of the various aspects and illustrative embodiments, it should be understood that many features are common to many aspects and embodiments. Omission of an aspect from a description or figure does not imply that the aspect is missing from embodiments that incorporate that aspect. Instead, the aspect may have been omitted for clarity and to avoid prolix description. In this context, the following applies to the rest of this description: If, in order to clarify the drawings, a figure contains reference signs which are not explained in the directly associated part of the description, then it is referred to previous or following description sections. Further, for reason of lucidity, if in a drawing not all features of a part are provided with reference signs it is referred to other drawings showing the same part. Like numbers in two or more figures represent the same or similar elements.

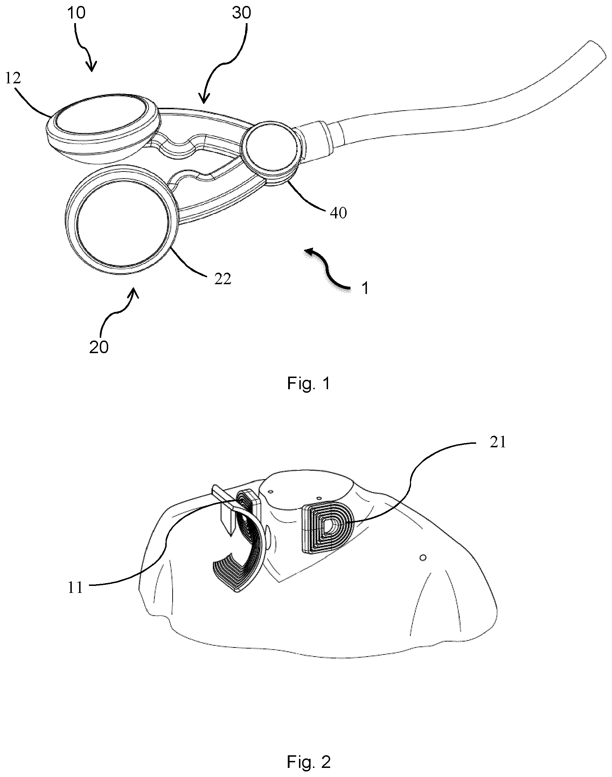

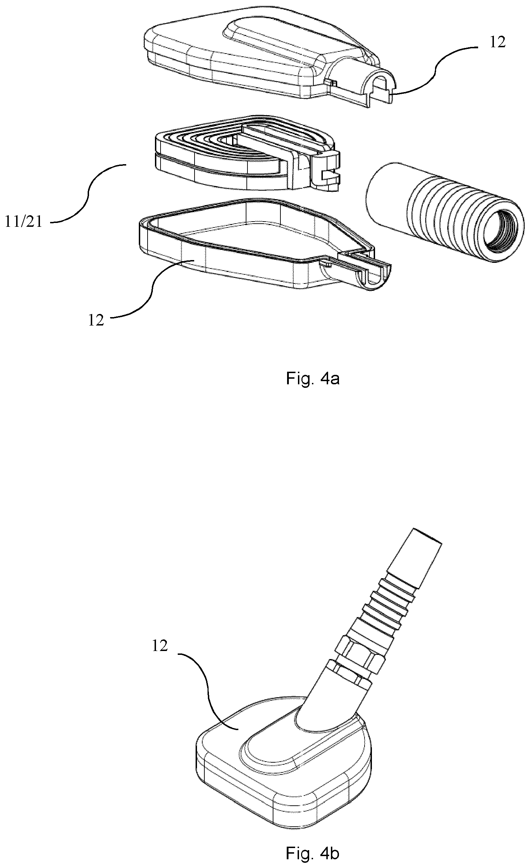

Fig. 1 illustrates an exemplary embodiment of a stimulation device 1 according to the invention. The stimulation device 1 is designed to stimulate a first and a second Phrenic nerve in a human body in a coordinated manner, thereby activating a diaphragm as target tissue in the human body. The stimulation device 1 comprises a first coil unit 10 and a second coil unit 20 that are electrically connected in series via a connector 40. In Fig. 1, coil units 10, 20 are schematically depicted whereas the more detailed shape and configuration thereof are depicted in Figs. 4a through 5c and described in the associated description below.

The first coil unit 10 comprise first two coil windings 11 as described below for generating a first electro-magnetic field to stimulate the first Phrenic nerve, and the second coil unit 20 comprises second two coil windings 21 as described below for generating a second electro-magnetic field to stimulate the second Phrenic nerve. The first and second coil units 10, 20 further have first and second housings 12 as described below in which the first and the second coil windings 11, 21 are arranged, e.g. enclosed, respectively.

In order that the stimulation device can be used at different sized bodies, the first and second coil units 10, 20 can be individually positioned at the body. In particular, for that purpose, the stimulation device 1 has a bracket structure 30 as support which mechanically couples the first 10 and second coil unit 20. As shown in Fig. 1, the first 10 and the second coil unit 20 are mechanically connected with each other only via the bracket structure 30. More specifically, the bracket structure 30 comprises two arms connected at one of their longitudinal ends via a joint member 40. An angle between the arms is adjustable at the joint member 40. At longitudinal end opposite to the joint member 40, the first and second coil units 10, 20 are pivotably mounted to the arms of the bracket structure 30. This facilitates the individual positioning of the coil units 10, 20 at the body of the human and, in particular, at a neck of the human.

Fig. 2 shows the two first coil windings 11 of the first coil unit 10 and the two second coil windings 21 of the second coil unit 20 placed at the neck of the human. The two coil windings 11, 21 of each of the first and second coil units 10, 20 are stacked on top of each other and formed in a D-shape such that they establish a straight-lined side 111, 211. More specifically, the straight-lined sides 111, 211 of the first and second coil windings 11, 21 extend along the neck and, thus, are parallel to each other. The stacked design of the two coil windings 11, 21 allow to increase the strength of the electro-magnetic field created.

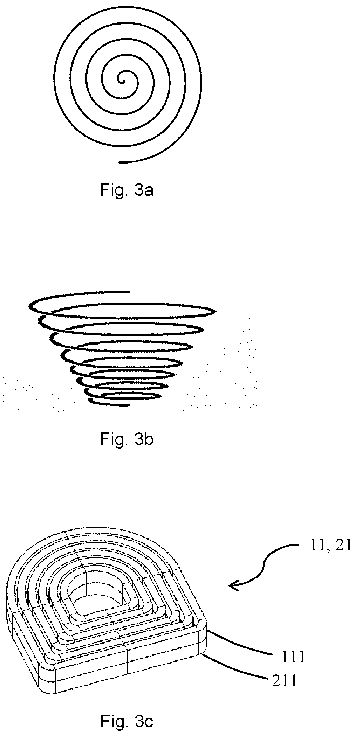

In Fig. 3a a general coil wound with varying radius into a spiral is shown. In particular, the radius of the spiral increases when moving from the centre of the spiral towards its periphery. The winding has flattened or pancake form, i.e. the coil winding quasi is in a plane. Fig. 3b shows a coil wound with varying radius into a conic form, i.e. not being in a plane.

Fig. 3c shows the two coil windings 11, 21 in the D-shaped form and stacked on top of each other. The two coil windings 11, 21 are wound with varying radius in a flattened form and consisting of one single litz wire. The D-shaped form of the spiral is particularly useful for stimulation of the Phrenic nerves since the electro-magnetic field generated has comparably high electric field gradient. Thus, the centre of the stimulation is close to the edge of the respective coil windings 11, 21, thereby closer to the neck of the human. It can be also easily tilted or rotated by the straight-lined side 111, 211 of the respective coil windings 11, 21. Since the two coil windings 11, 21 are stacked on top of each other, the size can be small in comparison with a single coil winding configuration. Accordingly, the stimulation can be performed more effectively and provides more convenience for the human when arranged at his neck.

Fig. 4a shows an exploded view of one of the coil units 10, 20 with the housing 12 and the two coil windings 11, 21. As shown the two coil windings 11, 21 are form-fit with the housing 12, which allows to reduce the size of the coil unit 10, 20. Fig. 4b shows the coil units 10, 20 when the housing 12 is closed. This is the normal situation when being placed at the neck of the human.

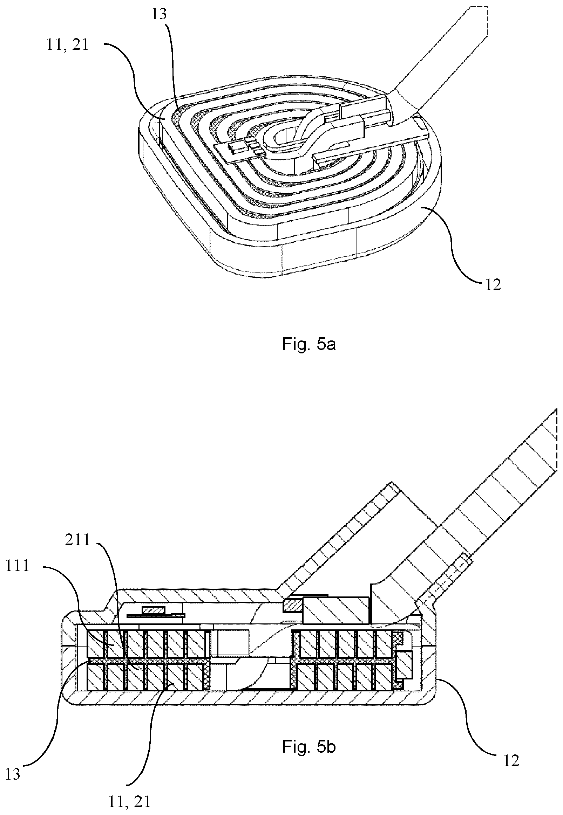

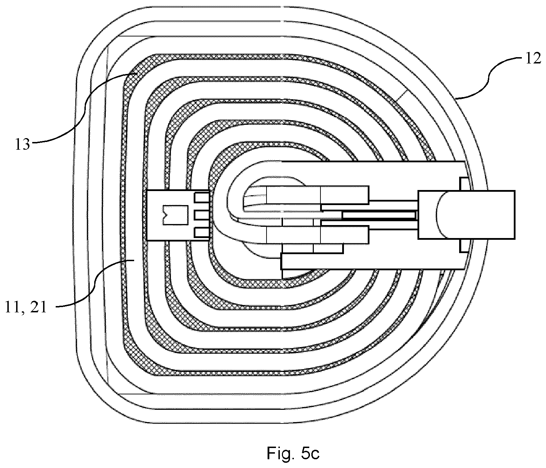

Fig. 5a to 5c show the different view of the housing 12 with the coil windings 11, 21 and an isolation member 13. As shown the coil windings 11, 21 are arranged form-fit in the housing and electrically isolated by the isolation member 13. At the same time, the isolation member 13 also a retaining or holding function for the coil windings 11, 21 and allows for efficiently wind the litz wire when creating the windings 11, 21.

This description and the accompanying drawings that illustrate aspects and embodiments of the present invention should not be taken as limiting-the claims defining the protected invention. In other words, while the invention has been illustrated and described in detail in the drawings and foregoing description, such illustration and description are to be considered illustrative or exemplary and not restrictive. Various mechanical, compositional, structural, electrical, and operational changes may be made without departing from the spirit and scope of this description and the claims. In some instances, well-known circuits, structures and techniques have not been shown in detail in order not to obscure the invention. Thus, it will be understood that changes and modifications may be made by those of ordinary skill within the scope and spirit of the following claims. In particular, the present invention covers further embodiments with any combination of features from different embodiments described above and below.

The disclosure also covers all further features shown in the Figs. individually although they may not have been described in the afore or following description. Also, single alternatives of the embodiments described in the figures and the description and single alternatives of features thereof can be disclaimed from the subject matter of the invention or from disclosed subject matter. The disclosure comprises subject matter consisting of the features defined in the claims or the exemplary embodiments as well as subject matter comprising said features.

Furthermore, in the claims the word "comprising" does not exclude other elements or steps, and the indefinite article "a" or "an" does not exclude a plurality. A single unit or step may fulfil the functions of several features recited in the claims. The mere fact that certain measures are recited in mutually different dependent claims does not indicate that a combination of these measures cannot be used to advantage. The terms "essentially", "about", "approximately" and the like in connection with an attribute or a value particularly also define exactly the attribute or exactly the value, respectively. The term "about" in the context of a given numerate value or range refers to a value or range that is, e.g., within 20%, within 10%, within 5%, or within 2% of the given value or range. Components described as coupled or connected may be electrically or mechanically directly coupled, or they may be indirectly coupled via one or more intermediate components. Any reference signs in the claims should not be construed as limiting the scope.

## Claims

1. A stimulation device (1) configured to stimulate a first Phrenic nerve and a second Phrenic nerve in a human or animal body for activating a target tissue such as a diaphragm in the human or animal body, comprising:
a first coil unit (10) configured to be positioned at the human or animal body to stimulate the first Phrenic nerve by an electric or electro-magnetic field generated by the first coil unit (10), and
a second coil unit (20) configured to be positioned at the human or animal body to stimulate the second Phrenic nerve by an electric or electro-magnetic field generated by the second coil unit (20),
wherein each of the first and second coil units (10, 20) comprises at least two coil windings (11, 21) being wound with a variable radius into a spiral and being stacked on top of each other.

2. The stimulation device (1) according to any one of the preceding claims, wherein the at least two coil windings (11, 21) of each of the first and second coil units (10, 20) comprises has at least one essentially straight-lined side (111, 211).

3. The stimulation device (1) according to claim 2, wherein the at least two coil windings (11, 21) of each of the first and second coil units (10, 20) are D-shaped or teardrop-shaped.

4. The stimulation device (1) according to claim 2, wherein the at least two coil windings (11, 21) of each of the first and second coil units (10, 20) have two straight-lined sides (111, 211), wherein the two straight-lined sides (111, 211) of the at least two coil windings (11, 21) of each of the first and second coil units (10, 20)
preferably are substantially parallel to each other,
preferably are parallel to each other and not in one plane, and/or
preferably have a length that is greater than a half of the equivalent spherical diameter of the at least two coil windings (11, 21).

5. The stimulation device (1) according to any one of the preceding claims, wherein the at least two coil windings (111, 211) of the first coil unit (10) are wound in a first direction, the at least two coil windings (11, 21) of the second coil unit (20) are wound in a second direction, and the first direction is opposite to the second direction.

6. The stimulation device (1) according to any one of the preceding claims, wherein each of the first and second coil units (10, 20) comprises a housing (12) for embracing the respective at least two coil windings (11, 21), wherein each housing (12) of the first and second coil units (10, 20) preferably comprises a spiral isolation member (13) configured to hold the respective at least two coil windings (11, 21) electrically isolated from each other.

7. The stimulation device (1) according to any one of the preceding claims, wherein the at least two coil windings (11, 21) of the first and second coil units (10, 20) are electronically connected with each other in series.

8. The stimulation device (1) according to any one of the preceding claims, wherein the first and second coil units (10, 20) are individually positionable at the human or animal body and/or are substantially axially arranged.

9. The stimulation device (1) according to any one of the preceding claims, further comprising a control unit configured to initiate the stimulation in accordance with a predefined security policy including conditions to activate or prevent the stimulation.

10. The stimulation device (1) according to any one of the preceding claims, configured such that each of the first and second coil units (10, 20) generates biphasic impulses.

11. The stimulation device (1) according to any one of the preceding claims, wherein the at least two coil windings (11, 21) of each of the first and second coil units (10, 20) are formed by the same litz wire and/or have an essentially identical form.

12. The stimulation device (1) according to any one of the preceding claims, wherein each of the at least two coil windings (11, 21) of each of the first and second coil units (10, 20)
is essentially flat,
is non-flat such as conical or bent, or
is bent in a radius between 4 cm and 20 cm, or between 6 cm and 12 cm.

13. The stimulation device (1) according to any one of the preceding claims, configured to generate time-variant currents in the first and second coil units (10, 20) being in timely synchrony.

14. The stimulation device (1) according to any one of the preceding claims,
comprising a stimulator unit configured to induce currents in the first and second coil units (10, 20),
configured such that an induced current in the first coil unit (10) is the same as an induced current in the second coil unit (20), and/or
configured to induce impulses generated by one current.

15. The stimulation device (1) according to any one of the preceding claims, wherein the first and second coil units (10, 20) have two forward faces
essentially opposing each other or opposing each other in an angle of +-20° or less, and/or
opposing each other +/- 20°.

## Patentansprüche

1. Stimulationsvorrichtung (1), ausgebildet zur Stimulation eines ersten Nervus phrenicus und eines zweiten Nervus phrenicus im menschlichen oder tierischen Körper zur Aktivierung eines Zielgewebes, wie beispielsweise eines Zwerchfells, im menschlichen oder tierischen Körper, umfassend:
eine erste Spuleneinheit (10), die dazu ausgebildet ist, am menschlichen oder tierischen Körper positioniert zu werden, um den ersten Nervus phrenicus durch ein von der ersten Spuleneinheit (10) erzeugtes elektrisches oder elektromagnetisches Feld zu stimulieren, und
eine zweite Spuleneinheit (20), die dazu ausgebildet ist, am menschlichen oder tierischen Körper positioniert zu werden, um den zweiten Nervus phrenicus durch ein von der zweiten Spuleneinheit (20) erzeugtes elektrisches oder elektromagnetisches Feld zu stimulieren,
wobei sowohl die erste als auch die zweite Spuleneinheit (10, 20) wenigstens zwei Spulenwicklungen (11, 21) umfasst, die mit variablem Radius spiralförmig gewickelt und übereinander gestapelt sind.

2. Stimulationsvorrichtung (1) nach einem der vorstehenden Ansprüche, wobei die mindestens zwei Spulenwicklungen (11, 21) sowohl der ersten als auch der zweiten Spuleneinheit (10, 20) wenigstens eine im Wesentlichen geradlinige Seite (111, 211) umfassen.

3. Stimulationsvorrichtung (1) nach Anspruch 2,
wobei die wenigstens zwei Spulenwicklungen (11, 21) sowohl der ersten als auch der zweiten Spuleneinheit (10, 20) D-förmig oder tropfenförmig sind.

4. Stimulationsvorrichtung (1) nach Anspruch 2,
wobei die wenigstens zwei Spulenwicklungen (11, 21) sowohl der ersten als auch der zweiten Spuleneinheit (10, 20) jeweils zwei geradlinige Seiten (111, 211) aufweisen, wobei die beiden geradlinigen Seiten (111, 211) der wenigstens zwei Spulenwicklungen (11, 21) sowohl der ersten als auch der zweiten Spuleneinheit (10, 20)
vorzugsweise im Wesentlichen parallel zueinander sind,
vorzugsweise parallel zueinander sind und nicht in einer Ebene liegen und/oder
vorzugsweise eine Länge aufweisen, die größer ist als die Hälfte des äquivalenten sphärischen Durchmessers der wenigstens zwei Spulenwicklungen (11, 21).

5. Stimulationsvorrichtung (1) gemäß einem der vorstehenden Ansprüche,
wobei die wenigstens zwei Spulenwicklungen (111, 211) der ersten Spuleneinheit (10) in einer ersten Richtung gewickelt sind, die wenigstens zwei Spulenwicklungen (11, 21) der zweiten Spuleneinheit (20) in einer zweiten Richtung gewickelt sind und die erste Richtung der zweiten Richtung entgegengesetzt ist.

6. Stimulationsvorrichtung (1) nach einem der vorstehenden Ansprüche,
wobei sowohl die erste als auch die zweite Spuleneinheit (10, 20) ein Gehäuse (12) umfasst, um die jeweiligen wenigstens zwei Spulenwicklungen zu umgeben (11, 21), wobei jedes Gehäuse (12) sowohl der ersten als auch der zweiten Spuleneinheit (10, 20) vorzugsweise ein spiralförmiges Isolationselement (13) umfasst, das dazu ausgebildet ist, die jeweiligen wenigstens zwei Spulenwicklungen (11, 21) elektrisch voneinander isoliert zu halten.

7. Stimulationsvorrichtung (1) nach einem der vorstehenden Ansprüche,
wobei die wenigstens zwei Spulenwicklungen (11, 21) der ersten und zweiten Spuleneinheit (10, 20) elektronisch in Reihe miteinander verbunden sind.

8. Stimulationsvorrichtung (1) nach einem der vorstehenden Ansprüche, wobei die erste und die zweite Spuleneinheit (10, 20) einzeln am menschlichen oder tierischen Körper positionierbar sind und/oder im Wesentlichen axial angeordnet sind.

9. Stimulationsvorrichtung (1) nach einem der vorstehenden Ansprüche,
ferner umfassend eine Steuereinheit, die dazu ausgebildet ist, die Stimulation gemäß einer vordefinierten Sicherheitsrichtlinie zu initiieren, welche Bedingungen zum Aktivieren oder Verhindern der Stimulation umfasst.

10. Stimulationsvorrichtung (1) nach einem der vorstehenden Ansprüche,
welche derart ausgebildet ist, dass sowohl die erste als auch die zweite Spuleneinheit (10, 20) biphasische Impulse erzeugt.

11. Stimulationsvorrichtung (1) nach einem der vorstehenden Ansprüche,
wobei die wenigstens zwei Spulenwicklungen (11, 21) sowohl der ersten als auch der zweiten Spuleneinheit (10, 20) aus demselben Litzendraht gebildet sind und/oder eine im Wesentlichen identische Form aufweisen.

12. Die Stimulationsvorrichtung (1) nach einem der vorstehenden Ansprüche,
wobei jede der wenigstens zwei Spulenwicklungen (11, 21) sowohl der ersten als auch der zweiten Spuleneinheit (10, 20)
im Wesentlichen eben ist,
nicht eben ist, wie etwa konisch oder gekrümmt, oder
in einem Radius zwischen 4 cm und 20 cm oder zwischen 6 cm und 12 cm gekrümmt ist.

13. Stimulationsvorrichtung (1) nach einem der vorstehenden Ansprüche,
welche dazu ausgebildet ist, zeitvariable Ströme in der ersten und der zweiten Spuleneinheit (10, 20) zu erzeugen, welche zeitlich synchron sind.

14. Stimulationsvorrichtung (1) nach einem der vorstehenden Ansprüche, umfassend eine Stimulator-Einheit, die dazu ausgebildet ist, Ströme in der ersten und in der zweiten Spuleneinheit (10, 20) zu induzieren,
derart ausgebildet ist, dass ein induzierter Strom in der ersten Spuleneinheit (10) der Gleiche ist wie ein induzierter Strom in der zweiten Spuleneinheit (20), und/oder
dazu ausgebildet ist, durch einen Strom erzeugte Impulse zu induzieren.

15. Stimulationsvorrichtung (1) nach einem der vorstehenden Ansprüche,
wobei die erste und die zweite Spuleneinheit (10, 20) zwei vorwärtige Seiten aufweisen,
welche im Wesentlichen einander gegenüberliegen oder in einem Winkel von ± 20° oder weniger einander gegenüberliegen, und/oder
die einander in einem Winkel von ± 20° gegenüberliegen.

## Revendications

1. Dispositif de stimulation (1) configuré à stimuler un premier nerf phrénique et un deuxième nerf phrénique dans le corps humain ou animal afin d'activer un tissu cible tel que le diaphragme dans le corps humain ou animal, comprenant :
une première unité de bobine (10) configurée pour être positionnée sur le corps humain ou animal afin de stimuler le premier nerf phrénique par un champ électrique ou électromagnétique généré par la première unité de bobine (10), et
une deuxième unité de bobine (20) configurée pour être positionnée sur le corps humain ou animal afin de stimuler le deuxième nerf phrénique par un champ électrique ou électromagnétique généré par la deuxième unité de bobine (20),
dans lequel chacune des première et deuxième unités de bobines (10, 20) comprend au moins deux enroulements de bobine (11, 21) enroulés avec un rayon variable en spirale et empilés l'un sur l'autre.

2. Dispositif de stimulation (1) selon l'une quelconque des revendications précédentes,
dans lequel les au moins deux enroulements de bobine (11, 21) de chacune des première et deuxième unités de bobine (10, 20) comportent au moins un côté essentiellement rectiligne (111, 211).

3. Dispositif de stimulation (1) selon la revendication 2,
dans lequel les au moins deux enroulements de bobine (11, 21) de chacune des première et deuxième unités de bobine (10, 20) sont en forme de D ou en forme de goutte.

4. Dispositif de stimulation (1) selon la revendication 2,
dans lequel les au moins deux enroulements de bobine (11, 21) de chacune des première et deuxième unités de bobines (10, 20) comportent deux côtés rectilignes (111, 211), dans lequel les deux côtés rectilignes (111, 211) des au moins deux enroulements de bobine (11, 21) de chacune des première et deuxième unités de bobines (10, 20)
sont de préférence sensiblement parallèles l'un à l'autre,
sont de préférence parallèles l'un à l'autre et ne se trouvent pas dans un même plan, et/ou
ont de préférence une longueur supérieure à la moitié du diamètre sphérique équivalent des au moins deux enroulements de bobine (11, 21).

5. Dispositif de stimulation (1) selon l'une quelconque des revendications précédentes,
dans lequel les au moins deux enroulements de bobine (111, 211) de la première unité de bobine (10) sont enroulés dans une première direction, les au moins deux enroulements de bobine (11, 21) de la deuxième unité de bobine (20) sont enroulés dans une deuxième direction, et la première direction est opposée à la deuxième direction.

6. Dispositif de stimulation (1) selon l'une quelconque des revendications précédentes,
dans lequel chacune des première et deuxième unités de bobines (10, 20) comprend un boîtier (12) destiné à renfermer les au moins deux enroulements de bobine respectifs (11, 21), dans lequel chaque boîtier (12) des première et deuxième unités de bobines (10, 20) comprend de préférence un élément d'isolation en spirale (13) configuré pour maintenir les au moins deux enroulements de bobine respectifs (11, 21) isolés électriquement les uns des autres.

7. Dispositif de stimulation (1) selon l'une quelconque des revendications précédentes,
dans lequel les au moins deux enroulements de bobine (11, 21) des première et deuxième unités de bobine (10, 20) sont connectés électroniquement entre eux en série.

8. Dispositif de stimulation (1) selon l'une quelconque des revendications précédentes,
dans lequel les première et deuxième unités de bobines (10, 20) peuvent être positionnées individuellement sur le corps humain ou animal et/ou sont agencées de manière sensiblement axiale.

9. Dispositif de stimulation (1) selon l'une quelconque des revendications précédentes,
incluant en outre une unité de commande configurée pour déclencher la stimulation conformément à une politique de sécurité prédéfinie comprenant des conditions pour activer ou empêcher la stimulation.

10. Dispositif de stimulation (1) selon l'une quelconque des revendications précédentes,
configuré de telle sorte que chacune des première et deuxième unités de bobines (10, 20) génère des impulsions biphasiques.

11. Dispositif de stimulation (1) selon l'une quelconque des revendications précédentes,
dans lequel les au moins deux enroulements de bobine (11, 21) de chacune des première et deuxième unités de bobine (10, 20) sont formés par le même fil Litz et/ou ont une forme essentiellement identique.

12. Dispositif de stimulation (1) selon l'une quelconque des revendications précédentes,
dans lequel chacun des au moins deux enroulements de bobine (11, 21) de chacune des première et deuxième unités de bobine (10, 20)
est essentiellement plat,
n'est pas plat, par exemple conique ou courbé, ou
est courbé selon un rayon compris entre 4 cm et 20 cm, ou entre 6 cm et 12 cm.

13. Dispositif de stimulation (1) selon l'une quelconque des revendications précédentes,
configuré pour générer des courants variant dans le temps dans les première et deuxième unités de bobines (10, 20) en synchronisation temporelle.

14. Dispositif de stimulation (1) selon l'une quelconque des revendications précédentes,
comprenant une unité de stimulation configurée pour induire des courants dans les première et deuxième unités de bobines (10, 20),
configurée de telle sorte qu'un courant induit dans la première unité de bobine (10) soit identique à un courant induit dans la deuxième unité de bobine (20), et/ou
configurée pour induire des impulsions générées par un courant.

15. Dispositif de stimulation (1) selon l'une quelconque des revendications précédentes,
dans lequel les première et deuxième unités de bobines (10, 20) présentent deux faces avant
essentiellement opposées l'une à l'autre ou opposées l'une à l'autre selon un angle de ± 20° ou moins, et/ou
opposées l'une à l'autre de ± 20°.
